# EUROPEAN PATENT SPECIFICATION

(11) **EP 3 638 219 B1**
(45) Date of publication and mention of the grant of the patent: **17.04.2024**
(21) Application number: 18732011.4
(22) Date of filing: 12.06.2018
(51) Int. Cl.: A61K 31/045, A61P 33/14, A61P 27/02, A61K 47/00, A61K 9/00

(54) **COMPOSITION COMPRISING TERPINENE-4-OL FOR THE TREATMENT OF DEMODICOSIS**
ZUSAMMENSETZUNG ENTHALTEND TERPINEN-4-OL ZUR BEHANDLUNG VON DEMODIKOSE
COMPOSITION COMPRENANT LE TERPINEN-4-OL POUR LE TRAITEMENT DE LA DÉMODÉCIE

(30) Priority: 12.06.2017 EP 17175567
(43) Date of publication of application: 22.04.2020
(73) Proprietor: NOVAX PHARMA S.A.M., 98000 Monaco (MC)
(72) Inventor: DIAS FERREIRA, Victor, 98000 Monaco (MC)
(74) Representative: Benedetto, Marco
(86) International application number: PCT/EP2018/065564
(87) International publication number: WO 2018/229084

(56) References cited:
- WO-A1-2012/061887
- Anonymous: "Eyevitality.com CLIRADEX PREMOISTENED MEDICATED LID SCRUB PADS - 24 PACK BOX - Eyelid Disease", , 1 January 2016 (2016-01-01), XP055428284, Retrieved from the Internet: URL:https://www.eyevitality.com/eyelid-dis ease/cliradex-premoistened-medicated-lid-s crub-pads-24-pack-box.html [retrieved on 2017-11-23]
- ANNY M.S. CHENG ET AL: "Recent advances on ocular Demodex infestation :", CURRENT OPINION IN OPHTHALMOLOGY, vol. 26, no. 4, 1 July 2015 (2015-07-01), pages 295-300, XP055371885, US ISSN: 1040-8738, DOI: 10.1097/ICU.0000000000000168
- SEAN TIGHE ET AL: "Terpinen-4-ol is the Most Active Ingredient of Tea Tree Oil to Kill Demodex Mites", TRANSLATIONAL VISION SCIENCE & TECHNOLOGY, vol. 2, no. 7, 1 November 2013 (2013-11-01), page 2, XP055372006, DOI: 10.1167/tvst.2.7.2 cited in the application

## Description

### Technical field of the invention

The present invention relates generally to a composition useful for the prevention and treatment of disorders of the external portion of the eye, in particular of blepharitis.

### Background of the invention

The present invention id defined in the appended claims.

The term "blepharitis" refers to inflammation of the eyelid. It is a common ocular disorder affecting several subjects and it may result from various inflammatory disease processes of the eyelid.

Blepharitis may be divided into anterior and posterior blepharitis. The anterior blepharitis refers to an inflammation around the eyelashes and follicles, while the posterior blepharitis involves the meibomian glands. The pathophysiology of blepharitis involves bacterial colonization of the eyelids, which in turn results in an immune-mediated inflammatory damage to the surrounding tissues.

Blepharitis is not usually a serious condition. However, a specific treatment is needed if you want to make the eye more comfortable. In a very few people the blepharitis can be severe and dangerous because it may damage the eyelids, and treatment can prevent more damage occurring.

The causes of blepharitis differ by type, whether acute or chronic, and for the latter cases, by whether it affects the outside or the underside of the eyelids. The underlying reason for the inflammatory condition is not understood, with the "cause" relating instead to observations of the disease processes.

Acute blepharitis can be ulcerative or non-ulcerative:
▪ Ulcerative acute blepharitis is usually caused by an infection - usually bacterial and usually staphylococcal. A viral cause is also possible, such as infection with *Herpes simplex* and *Varicella zoster.*
▪ Non-ulcerative acute blepharitis is usually an allergic reaction. Examples of this include atopic blepharo-dermatitis, seasonal allergic blepharo-conjunctivitis, and dermato-blepharo-conjunctivitis.

The diagnosis of blepharitis is usually concerned with the chronic forms. Although academic descriptions classify up to six specific forms of chronic blepharitis, for practical purposes it is enough to look at two broad types according to the part of the eyelids affected:
▪ In anterior blepharitis affecting the outer part of the front of the eyelids, a bacterial (usually staphylococcal) or seborrheic disease process is involved. Seborrhea is poorly understood; it manifests as dandruff when it affects the scalp. Patients with anterior blepharitis often also have seborrheic dermatitis of the face and scalp, or another skin condition called acne rosacea.
▪ In posterior blepharitis affecting the underside of the front of the eyelids, there is a problem with the glands near the base of the eyelashes (the condition is also known as Meibomian gland dysfunction, MGD). The glands over-secrete their oily substance or become blocked. The reason for this is not well understood, although hormonal causes are suspected and the condition is often associated with acne rosacea. Posterior blepharitis can also be associated with seborrheic dermatitis.

Symptoms of blepharitis include burning, watery eyes, foreign body sensation, red eyelids, red eyes, pain and blurry vision. A physical examination of patients with blepharitis can often show a loss of eyelashes, the plugging of the meibomian glands, and/or infection of the conjunctiva. Also, the irritation caused by blepharitis is often a cause of eye dryness. In case of severe blepharitis, the cornea can be affected, causing marginal infiltrates, marginal ulcers, and pannus formation.

Use of heavy cosmetics may give rise to the symptoms of blepharitis. In such situations, even though the patients are advised to stop wearing the cosmetics, they typically refuse to do so, in order to maintain their desired appearance, and worsening of blepharitis can occur.

The main features of blepharitis are the following problems with the eyelids: Itchiness, Redness, Flaking and Crusting.

The crusting may be more prominent on waking in the morning - all of the symptoms generally tend to be worse at this time.

Complaints also include:
Sensations of burning, itching, irritation or that there is a foreign body or a grittiness in the eyes.
Excessive eye watering, tears (epiphora, excessive over-production of tears).
Conversely, dry eyes (especially with posterior blepharitis).
Stickiness of the eyes.
Oversensitivity to light (photophobia).
Intolerance to wearing contact lenses.

Blepharitis is not a sight-threatening problem, but it can result in decreased vision, which can come and go.

The condition usually affects both eyes, it is bilateral - and even if it is present in only one eye, this can lead a physician to suspect an alternative diagnosis.

The symptoms of chronic blepharitis tend to come and go, with periods of remission (a pause in symptoms) followed by exacerbations (a flare up).

Mixed blepharitis is seborrheic with associated staph. Patients who suffer from blepharitis as a result of an ocular demodex mites infestation often refer a number of symptoms such as a foreign body sensation, redness and itching. The symptoms can become severe enough that the patient may require surgery to achieve relief.

However, even invasive treatments such as surgery often result in no significant improvement, and/or a recurrence of the demodex mites infestation.

Demodex mites (hereafter: "demodex"), class Arachnid and order Acarina, are elongated ectoparasites. Among a wide range of reported species, only two, *D. folliculorum* and *D. brevis*, are found on the human body surface and are the most commn ectoparasites infesting the pilosebaceous unit of the skin. Adult *D*. *folliculorum* is commonly found in small hair follicles. *D. brevis* burrows deeper into the eyelash sebaceous gland and the meibomian gland. Both demodex species often coexist at the same skin area and tend to gather in the face, cheeks, the forehead, the nose, and the external ear tract, where their habitat and breeding are favoured by active sebum excretion. Demodicosis, i.e. infestation of the skin by demodex, is believed to be the most common, although often overlooked, cause of up to 74% of cases of blepharitis.

The demodex mites can also infest mammalian quadrupeds, in particular domestic animals, especially dogs, causing demodetic mange, which can involve the face and the entire body of the animal in some cases. Existing treatments can be expensive and are not always effective, with the result that affected animals are sometimes euthanized. In general, parasitic mites such as those causing mange in pets are transmissible to humans.

Generally, demodex is currently treated with both systemic and topical administration of parasiticides. For example, ocular demodex in humans is treated by daily practice of eyelid margin scrub with diluted shampoo alone or combined with 1% mercury oxide ointment, 2% metronidazoles gel or Pilocarpine gel at the base of the eye lashes at the night time. Ophthalmic reports demonstrated a dramatic decrease in the demodex count observed during the follow-up examination.

However, to date, no report has demonstrated that these treatments actually kill demodex *in vitro* and no therapeutic agent has completely eradicated ocular demodex within one month. Indeed, recent investigations revealed that ocular demodex persists in 50% of patients even after one year of lid scrub with ordinary, diluted shampoo alone or combined with 1% mercury oxide ointment, 2% metronidazoles gel or Pilocarpine gel.

Therefore there is a strong need for formulations that may be useful for treating or preventing blepharitis, and in particular formulations that have anti-bacterial and anti-inflammatory properties without the side effects of traditional antiparasitic agents, which may also lead to allergic reactions in some subjects.

The patent applications WO06119174 A1 and WO09032773 A2 describe the use of a topical composition comprising tea tree oil (TTO), such as a TTO shampoo and a scrub, for the *in vivo* treatment of demodex mites infestation. Said patent applications WO06119174 A1 and WO09032773 A2 indicate that specific components of TTO, such as terpenen-4-ol and carvone, are active against demodex *in vitro.*

Tighe, S. et al (TVST 2013, vol 2, No. 7, Article 2, page 1-8) describe an *in vivo* demodex killing assay on a single patient using a lid cleanser containing terpinen-4 ol, water and minor ingredients to help solubilize the active ingredient and refer that terpinen-4-ol can have decreased solubility, hence lower potency, when mixed with other ingredients. Tighe *et al* also report that allergic reactions have been observed when TTO was used as topical formulation for skin treatment and that the adverse reactions have been linked to the oxidation products of TTO components.

Pharmaceutical preparations in the form of a foam are particularly suitable to apply to the eyelid area and result in a longer action than other forms, such as wipes and drops. For these reasons, it would be particularly advantageous to prepare a foam containing terpinen-4-ol, or TTO extracts, for topical administration.

The current inventors, however, have found that the preparation of topic formulations comprising said active ingredients of TTO is particularly challenging, due to poor stability and difficult foamability of the compositions, particularly those containing terpinen-4-ol.

The aim of the present invention is to provide a composition for use in the treatment and/or prevention of demodex mite infestation and demodex-related blepharitis which is stable, tolerable and without the drawbacks of the currently available medicaments.

The aim of the present invention is also to provide a composition for use in a method of treatment and/or prevention of demodex mite infestation and demodex-related blepharitis which is in an administration form suitable for being applied to the skin, dermis, epidermis and/or to the eyelid area. Suitable administration forms are represented by foam, wipes and drops.

The present invention provides a composition containing terpinen-4-ol in a pharmaceutical form or administration form which is suitable for topic administration in periocular region or on the skin, and which is stable, tolerable and can be easily applied.

The composition of the present invention is a pharmaceutical composition, a composition for a medical device or a cosmetic composition.

In one aspect, the present invention provides a composition comprising a mixture which comprises or, alternatively, consists of:
(a) terpinen-4-ol;
(b) a mixture comprising or alternatively consisting of caprylyl/capryl glucoside (b5), sodium cocoyl glutamate (b6), polyglyceryl-5 oleate (b7), glyceryl caprylate (b8), and
optionally comprising also cocamidopropyl betaine.

In one embodiment, the present invention relates to a pharmaceutical or cosmetic formulation comprising said composition, preferably in the form of a foamable composition, liquid composition or solid composition such as a wipe impregnated with said composition.

In another embodiment, the present invention relates to a method for the preparation of the composition as defined above, in the presence of cocamidopropyl betaine (c), comprising the steps of:
(i) mixing the components (a) and (b) in the presence of water;
(ii) adding under stirring cocamidopropyl betaine (c) to the mixture obtained by step (i).

The present invention further provides the composition as defined above for use in the treatment or the prevention of demodex infestation (demodicosis) and related diseases and conditions as defined in the claims in a subject, wherein the use comprises the topical administration of said composition to a part of a subject affected by demodicosis. Preferably, the physical parts of the subject affected by demodicosis eyelid, eyelashes and eye contour.

The composition of the present invention can be used for the treatment of demodex in humans or in animals, such as pets.

In the context of the present invention, the content of an ingredient in a mixture refers to the weight of the ingredient with respect to the total weight of the mixture, unless otherwise specified.

In the context of the present invention, the indication that a composition "comprises" a certain component, or a mixture of components, does not exclude that said composition may include other components, in addition to that or those explicitly mentioned.

In the context of the present invention the terms formulation and composition are used interchangeably when it is not otherwise disclosed.

The inventors found that the formulations of terpinen-4-ol disclosed in the prior art are unstable, likely due to the incompatibility of the active ingredient with some other ingredients and excipients, for unknown causes, and to the tendency of terpinen-4-ol to degrading over time.

In particular, it was found that terpinen-4-ol, also in concentrations below 2% in weight/weight of the composition, tends to separate from aqueous media, so as to form a two-phase system, even if it is in the presence of commonly used emulsifiers and surfactants.

The present inventors found that commonly used surfactants, despite having high HLB (hydrophilic-lipophilic balance) number, do not provide a stable solution of terpinen-4-ol in aqueous media. For example, a two-phase system was obtained in the presence of Tween^{®} 20 in a weight ratio to terpinen-4-ol up to 5:1.

At the end of an extensive research activity, it was found that stable monophasic systems comprising terpinen-4-ol in aqueous media could be obtained by using specific additives, namely at least one from polysorbate 80 (b1), PPG-26 buteth-26 (b2), PEG-40 hydrogenated castor oil (b3), trideceth-9 (b4), caprylyl/capryl glucoside (b5), sodium cocoyl glutamate (b6), polyglyceryl-5 oleate (b7), glyceryl caprylate (b8), polyglyceryl-4-caprylate (b9), decyl glucoside glutamate (b10), diglycerin (b11) and mixtures thereof.

The compositions according to the invention are physically stable, i.e. clear, transparent and without precipitates, and substantially retain the initial content of the initial ingredient.

The composition of the present invention comprises caprylyl/capryl glucoside (b5), sodium cocoyl glutamate (b6), polyglyceryl-5 oleate (b7), glyceryl caprylate (b8).

According to the invention, the composition comprises ingredient (b) that is a mixture comprising or, alternatively, consisting of caprylyl/capryl glucoside (b5), sodium cocoyl glutamate (b6), polyglyceryl-5 oleate (b7), glyceryl caprylate (b8).

Polysorbate 80 (b1) (CAS number 9005-65-6) is a synthetic non-ionic surfactant and emulsifier, often used in foods and cosmetics, which is a viscous, water-soluble yellow liquid. The full chemical names for polysorbate 80 are Polyoxyethylene (20) sorbitan monooleate, or (x)-sorbitan mono-9-octadecenoate poly(oxy-1,2-ethanediyl). Polysorbate 80 is commercially available under various tradenames, such as Tween^{®} 80. Polysorbate 80 is commonly used as an emulsifier in foods and cosmetics and is used as a stabilizer for aqueous formulations of medicament for parenteral administration.

PPG-26 Buteth-26 (b2) (CAS number 9065-63-8 or 9038-95-3) is a synthetic polymer prepared from small chemical compounds called monomers, which is part of a class of polymers generally indicated as PPG Buteths (PEG/PPG butyl ethers, Polyethylene-polypropylene glycol, monobutyl ether). Said polymers are generally used in cosmetic formulations to enhance the appearance and feel of hair, by increasing hair body, suppleness, or sheen, or by improving the texture of hair that has been damaged physically or by chemical treatment. They also enhance the appearance of dry or damaged skin by reducing flaking and restoring suppleness. PPG Buteths help to form emulsions by reducing the surface tension of the substances to be emulsified.

PPG Buteths are made from equal amounts ethylene oxide and propylene oxide monomers, and the reaction is initiated by the addition of butyl alcohol. The first number in the name indicates the average number of units of propylene oxide, while the second number indicates the average number of units of ethylene oxide, hence PPG-26 Buteth-26 contain an average number of 26 propylene units and 26 ethylene units.

PEG 40 hydrogenated castor oil (b3) (CAS number 61788-85-0) is the polyethylene glycol derivative of hydrogenated castor oil, and is an amber colored, slightly viscous liquid that has a naturally mildly fatty odor. PEG Hydrogenated Castor Oils are produced from hydrogenated castor oil. PEG 40 hydrogenated castor oil has 40 mol EO average molar ratio. Castor oil is obtained by the cold pressing of seeds of the *Ricinus communis* plant followed by clarification of the oil by heat. It is used in cosmetics and beauty products as an emulsifier, surfactant, and fragrance ingredient.

Trideceth-9 (b4) (CAS number 38471-49-7 or 24938-91-8) is a polyethylene glycol ether of tridecyl alcohol, also indicated as PEG-9 tridecyl ether, polyoxyethylene(9)-tridecyl ether, tridecyl ethyleneglycol monoether) generally used as a surfactant and/or emulsifying agent in cosmetic formulations.

Preferably, the composition of the invention comprises water as a solvent, more preferably from 20% to 90%, even more preferably from 70% and 85%, of water in weight over the total weight of the composition.

The composition of the invention is stable also in case of high content or concentration of terpinen-4-ol.

In one embodiment of the invention, the amount of terpinen-4-ol is comprised from 0.01% to 10% by weight, with respect to the total weight of the composition, more preferably from 0.1% to 5% by weight.

In a preferred embodiment, the amount of terpinen-4-ol is from 0.1% to 10%, more preferably from 1% to 5% or 1% to 2.5% , in weight over the total weight of the composition.

In one embodiment of the invention, the weight ratio between ingredient (b) and terpinen-4-ol (a) is from 0.01:1 to 10:1.

In a preferred embodiment, the weight ratio between ingredient (b) and terpinen-4-ol (a) is from 0.1:1 to 10:1; preferably from 1: 1 to 7: 1, from 2:1 to 5:1 or from 3:1 to 4.5:1.

The composition according to the invention is suitable for the preparation of topical medications, such as a liquid pharmaceutical formulation or a liquid formulation for medical device, e.g. a solution, a suspension, a lotion, eye drops and similar forms, a semi-solid dosage, e.g. a cream, a gel, an ointment, a gel, a paste, a foam, or a solid formulation such as a tissue, a wipe, a scrub, a plaster, a patch and similar forms.

In particular, the composition according to the invention is suitable for being impregned by or absorbed on a solid substrate (e.g. a wipe or a tissue) when it is in the form or physical state of a liquid.

The composition according to the invention is in a form or physical state of a foam (foamable composition), liquid, solid or gel.

In a preferred embodiment, the composition according to the invention is used to impregnate a tissue or a wipe, that is suitable to be applied in the ocular area or, alternatively, on the skin, epidermis or dermis.

The inventors found that the following combinations of ingredients are particularly advantageous for the preparation of wipes or tissues containing the composition according to the invention, so as to obtain stability, availability and efficient delivery of the active principle terpinen-4-ol to the site of the intended antiparasitic action.

Preferably, the composition according to the invention is in the form of a tissue or wipe and comprises caprylyl/capryl glucoside (b5), sodium cocoyl glutamate (b6), polyglyceryl-5 oleate (b7), glyceryl caprylate (b8), or a mixture thereof.

In a preferred embodiment, the composition is in the form or physical state of a liquid and the at least one ingredient (b) is a mixture comprising or, alternatively, consisting of caprylyl/capryl glucoside (b5), sodium cocoyl glutamate (b6), polyglyceryl-5 oleate (b7), glyceryl caprylate (b8).

Said mixture (b) comprising caprylyl/capryl glucoside (b5), sodium cocoyl glutamate (b6), polyglyceryl-5 oleate (b7), glyceryl caprylate (b8) is, for example, available as commercial product commercialized with the name of Symbio^{®}solv clear by Dr. Straetmans GmbH. The composition of the commercial product Symbio^{®}solv clear is the following: D-Glucopyranose, oligomers, decyl octyl glycosides; Water; 1,2,3-Propanetriol, homopolymer, (Z)-9- octadecenoate; L-Glutamic acid, N-coco acyl derivs., monosodium salts; Octanoic acid, monoester with glycerol; Citric acid (INCI name: Caprylyl/Capryl Glucosides; Aqua; Polyglyceryl-5 Oleate; Sodium Cocoyl Glutamate; Glyceryl Caprylate; Citric acid; CAS: 500-220-1; 231-791 -2; Polymer; 269-087-2; 247-668-1 ; 201-069-1)

Caprylyl/capryl glucoside (b5) is an alkyl polyglucoside (CAS number 68515-73-1) and is a mild, solubilising non-ionic surfactant obtained from renewable raw materials: fatty alcohols and glucose from vegetable origin. Caprylyl/capryl glucoside.

Sodium cocoyl glutamate ((b6), CAS number 68187-32-6 or 68187-30-4) is mild, eco-friendly surfactant.

Polyglyceryl-5 oleate (b7) is an ester of oleic acid and a glycerin polymer containing an average of 5 glycerin units (CAS number 86529-98-8 or 9007-48-1) and is mainly used an environmentally friendly and mild surfactant.

Glyceryl caprylate (b8), CAS number 26402-26-6, is a non-ionic surfactant made from 100% renewable sources, through the esterification of glycerin from vegetable oil sources and medium chain fatty acids, for example of coconut and / or palm kernel oil.

Polyglyceryl-4-caprylate (b9) is a monoester of caprylic Acid and polyglycerol, generally used as solubilizer in cosmetic compositions to solubilize natural oils and lipophilic emollients, and 100% based on renewable raw materials.

Decyl glucoside glutamate (b10) (CAS number 113114-16-2) and diglycerin (b11) (a highly pure polyol with four hydroxyl groups produced through glycerin reaction, CAS number 59113-36-9) are also used as environmentally-friendly emulsifiers in cosmetic compositions.

A further object of the present invention is a solid substrate comprising the composition according to the present invention, wherein said composition in the form or physical state of a liquid is absorbed on or impregned by the solid substrate, preferably the solid substrate is a wipe or a tissue.

In a preferred embodiment, the solid substrate comprises the composition of the invention wherein said at least one ingredient (b) is a mixture comprising or, alternatively, consisting of caprylyl/capryl glucoside (b5), sodium cocoyl glutamate (b6), polyglyceryl-5 oleate (b7), glyceryl caprylate (b8), such as the commercial product Symbio^{®}solv clear described above.

The solid substrate of the invention is for use in a method of treatment or prevention of demodex infestation (demodicosis), demodicosis-related disease or demodicosis-related disorder in a subject in the need thereof, wherein said composition is used by topical administration.

In a preferred embodiment, the composition of the invention is in the form of a foamable composition or a foam.

In the context of the present invention, the term "foamable composition" indicates a mixture that is suitable to be transformed into a foam, for example upon addition of a blowing agent such as a substance which is a gas at the temperature at which the foam is formed or a substance capable of producing gas via a chemical reaction. Generally, such compositions are placed in a sealed contained, such as a conventional gas can, for dispensing upon actuation of a foam releasing device.

As non-limiting examples, chemical blowing agents can be isocyanate and water (for PUs), azo-, hydrazine and other nitrogen-based materials (for thermoplastic and elastomeric foams), sodium bicarbonate (aka baking soda, used in thermoplastic foams). Here gaseous products and other by-products are formed by a chemical reaction(s), promoted by process or a reacting polymer's exothermic heat. Since the blowing reaction occurs forming low molecular weight compounds acting as the blowing gas, additional exothermic heat is also released. Powdered titanium hydride is used as a foaming agent in the production of metal foams, as it decomposes to form hydrogen gas and titanium at elevated temperatures. Zirconium(II) hydride is used for the same purpose.

Once formed the low molecular weight compounds will never revert to the original blowing agent(s), i.e. the reaction is irreversible.

Alternatively, the composition according to the invention can be foamed by addition of air under moderate pressure, for example by a hand-operated foamer pump, as in a foamer bottle commonly used for dispensing soaps, pharmaceuticals and household products.

It was found that the addition of a surfactant such as an amphoteric and/or anionic substance can act as a "foam booster" to improve the release and/or the stability of the composition of the invention in the form of a foam, contributing to ease of application and enhancing the overall therapeutic effect of the medicament. Preferably, said amphoteric substance is a derivative of a dialkylaminoalkylamine, or lauramidopropyl betaine or 2-[3-(Dodecanoylamino)propyl(dimethyl) azaniumyl]-acetate) and/or the anionic substance is lauryl ether sulfate, lauryl ether sulfosuccinate, or mixtures thereof.

More preferably, said amphoteric substance is a cocamidopropyl derivative, such as cocamidopropyl betaine or hydroxysultaine. In a preferred embodiment, said amphoteric substance is cocamidopropyl betaine.

In a preferred embodiment, the composition according to the present invention further comprises a surfactant (c) selected from cocamidopropyl betaine, lauryl ether sulfate, lauryl ether sulfosuccinate, and mixtures thereof. In one embodiment, said surfactant (c) is present in the composition in an amount comprised from 1% to 25% by weight, with respect to the total weight of the composition; preferably in an amount from 8% to 15% by weight, more preferably in an amount from 5% to 20% by weight.

Cocamidopropyl betaine (CAPB, CAS number 61789-40-0) is a mixture of closely related organic compounds derived from coconut oil and dimethylaminopropylamine. CAPB is available as a viscous pale yellow solution and it is used as a surfactant in personal care products. The major part of the molecule, the lauric acid group, is derived from coconut oil. Cocamidopropyl betaine is used in cosmetics and personal care products, such as shampoo, as a medium strength surfactant.

Preferably, said amphoteric substance is present in the composition of the invention in an amount ranging from preferably in amount from 8% to 15%, more preferably from 10% to 12%, in weight over the total weight of the composition.

The composition according to the present invention can optionally comprise other ingredients such as buffering agents (e.g. tris-(hydroxymethyl) aminomethane, triethanolamine), antifibrotic agents (e.g. taurine), moisturizers (e.g. panthenol, allantoin, sodium hyaluronate), antimicrobial agents (.g. sodium hydroxymethyl glycinate), metal sequestering agents (e.g. EDTA), preservatives such as ethylhexylglycerin and phenethyl alcohol mixture, inorganic salts (e.g. sodium chloride), natural plant extracts (such as *Chamomila romana* extracts, *Aloe vera* or *barbadensis*), vitamins (e.g. vitamin E and its derivatives and provitamins, vitamin B and its derivatives and provitamins), hydrolysed or partially hydrolysed proteins, perfumes, amphiphilic surfactants and similar excipients as known to the person skilled in the art.

In a preferred embodiment, the composition according to the invention comprises hyaluronic acid, or its derivatives and salts, such as sodium salt, and/or polysaccharides from *Aloe vera* gel, glycosaminoglycans, polysaccharides from natural sources, e.g. from bacteria, algae or seaweed, xanthan gum, and mixtures thereof. More preferably, each of said ingredients is in a range from 0.01 to 2%, preferably from 0.05 to 1% in weight over the total weight of the composition.

Hyaluronic acid in the composition of the invention can be of high molecular weight (i.e. 2 × 10⁶ Da and above), medium molecular weight (8 × 10⁵ to 2 × 10⁶ Da) or low molecular weight (from 0.8 to 8 × 10⁵ Da).

In a preferable embodiment, the composition according to the invention comprises, in addition to (a) terpinen-4-ol and (b) a mixture comprising or, alternatively, consisting of caprylyl/capryl glucoside (b5), sodium cocoyl glutamate (b6), polyglyceryl-5 oleate (b7) and glyceryl caprylate (b8), hyaluronic acid or its salts, such as sodium salt, *Aloe vera* gel and panthenol or D-panthenol.

In a more preferable embodiment, the composition according to the invention is in the form or physical state of a liquid absorbed on or impregned by a solid substrate, such as a wipe or a tissue, and comprises, in addition to (a) terpinen-4-ol and (b) a mixture comprising or, alternatively, consisting of caprylyl/capryl glucoside (b5), sodium cocoyl glutamate (b6), polyglyceryl-5 oleate (b7) and glyceryl caprylate (b8), hyaluronic acid or its salts, such as sodium salt, *Aloe vera* gel and panthenol or D-panthenol.

In a further preferable embodiment, the composition according to the invention is in the form or physical state of a liquid absorbed on or impregned by a solid substrate, such as a wipe or a tissue, and it comprises in addition to (a) terpinen-4-ol and (b) a mixture comprising or, alternatively, consisting of caprylyl/capryl glucoside (b5), sodium cocoyl glutamate (b6), polyglyceryl-5 oleate (b7) and glyceryl caprylate (b8), and also hyaluronic acid or its salts, such as sodium salt, *Aloe vera* gel, panthenol or D-panthenol and ethylhexylglycerin and phenethyl alcohol mixture.

Said ethylhexylglycerin and phenethyl alcohol mixture is, for example, commercially available with the name Sensiva^{®}PA20 distributed by Schuelke (INCI name: ethylhexylglycerin, phenethyl Alcohol; CAS: 70445-33-9; 60-12-8).

### Comparative composition A Composition A (according to the invention)

| **Components** | **Amount (% in weight over total weight of composition)** |
|---|---|
| Water | Up to 100 |
| PEG-40 hydrogenated castor oil (b3), PPG-26 Buteth-26 (b2), | 1.0 -12.00 |
| Trideceth-9 (b4) | 0.1 to 5% |
| Terpinen-4-ol | up to 5% |
| Other ingredients: | |

Examples of the formulation (or composition) according to the present disclosure are the following:

### Comparative example 1 Formulation for 100 g:

| **Components** | **Amount (%)** |
|---|---|
| Water | Up to 100 |
| Panthenol | 1.00 |
| Allantoine and/or aloe | 0.05 - 1.5 |
| Taurine | 1.80 |
| Tris-tromethamine | 0.235 |
| Acid boric | 0.30 |
| Cocamidopropyl betaine | 5.00 - 15.00 |
| PEG-40 hydrogenated castor oil, PPG-26 Buteth-26, Trideceth-9 | 1.00 - 10.00/1.5 -15.00 |
| Terpinen-4-ol | 0.1-5.00 |
| *Chamaemelum nobile* essential oil | 0.10 |
| N-hydroxy methylglycinate sodium | 0.20 |
| EDTA | 0.10 |
| Helychrysum Glycolic extract | 0.20 |
| Aroma (* if present) | 0.10* |

### Example 2 (according to the claimed invention):

| **Components** | **Amount (%)** |
|---|---|
| Water | Up to 100 |
| Terpinen-4-ol | 1-5; 1.5-3.5 |
| Caprylyl/Capryl glucoside, sodium cocoyl glutamate, polyglyceryl-5-oleate, glyceryl caprylate (+citric acid) | 1-5; 2-3.5 |
| Ethylhexylglycerin and phenethyl alcohol mixture | 0.1-2; 0.3-1.5 |
| Triethanolamine | 0.1-1.5; 0.2-0.7 |
| Hyaluronic acid Na salt - high MW | 0.01-0.5; 0.02-0.3 |
| EDTA .2Na | 0.01-0.1;0.02-0.07 |
| *Aloe vera gel* 1-1 polysaccharides 10% | 0.01-2; 0.02-1.5 |
| Panthenol | 0.001-2.5; 0.05- |
| Vitamin E acetate | 1.50.001-0.9; 0.005-0.6 |

The cotton wipes of "STERILE WIPES with terpinen-4-ol, sodium hyaluronate and aloe vera" are soaked in the following solution:

| RAW MATERIALS | Functions | CAS number | % |
|---|---|---|---|
| PURIFIED WATER | Solvent | 7732-18-5 | 90-95 |
| EDTA SODIUM SALT | Chelating | 6381-92-6 | 0.01-0.10 |
| SENSIVA PA 20: Phenethyl Alcohol, Ethylhexylglycerin (stabilized with alpha-tocopherol) | Preservative Mild humectant agent | 60-12-8, 70445-33-9, 7695-91-2 | 0.05-1.5 |
| ALOE BARBADENSIS LEAF JUICE 10% POLYSACCHARIDES | Humectant and Lubricant agent | 85507-69-3 | 0.01-0.1 |
| PANTHENOL | Moisturizing | 81-13-0 | 0.0005-0.002 |
| SODIUM HYALURONIC ACID | Lubricant and Moisturizing agent | 9004-61-9 | 0.05-0.20 |
| TRIETHANOLAMINE 99% | pH balancer | 102-71-6 | 0.50-1.5 |
| SYMBIOSOLV CLEAR: Caprylyl/Capryl Glucoside; Aqua; Polyglyceryl-5 Oleate; Sodium Cocoyl Glutamate; Glyceryl Caprylate; Citric acid | Solubilizer | 68515-73-1, 7732-18-5, 86529-98-8, 68187-30-4, 26402-26-6, 77-92-9 | 2-3 |
| TOCOPHERYL ACETATE | Preservative | 7695-91-2 | 0.0005-0.003 |
| TERPINEN-4-OL | Preservative | 562-74-3 | 2-3 |

In an embodiment, the present invention relates to a method for the preparation of the composition as defined above, comprising the step of:
(i) mixing the components (a) and (b) in the presence of water or a water-miscible solvent.

Preferably, wherein the composition further comprises cocamidopropyl betaine (c), the method of the present invention thus further comprises the step of:
(ii) adding under stirring cocamidopropyl betaine (c) to the mixture obtained by step (i).

It was found that, for the preparation of a composition comprising cocamidopropyl betaine (c), the order of addition is important to ensure physical stability of the composition. In fact, upon mixing (a), (b) and cocamidopropyl betaine (c), generally an opalescent suspension is obtained, which eventually results in a dishomogeneous system. This is why the cocamidopropyl betaine (c) is added after having mixed the components (a) and (b) in the presence of water or a water-miscible solvent.

The inventors found that a clear and stable solution can be obtained following addition of cocamidopropyl betaine to a pre-formed mixture of ingredients (a) and (b). For example, on a lab scale, terpinen-4-ol and a mixture of PPG-26 buteth-26 (b2), PEG-40 hydrogenated castor oil (b3) and trideceth-9 (b4) were mixed in water and stirred for about 30 minutes. Cocamidopropyl betaine was then added and a clear solution was obtained, which was verified as stable.

Preferably, in the method of the invention, water or a water-miscible solvent in step (i) is present in an amount from 50 to 95% in weight over the total weight of the mixture of step (i).

In an embodiment, the present invention relates to a pharmaceutical or cosmetic formulation comprising the composition as defined above, preferably in the form of a foamable composition, a liquid composition or a solid composition such as a wipe impregnated with said composition.

Preferably, the formulation of the invention is in the form of a foamable composition, further comprising at least a foaming agent such as, but not limited to, a blowing agent selected from a gas and an agent capable of producing gas via a chemical reaction. Said formulation is preferably in a container suitable for dispensing a foam upon actuation of a suitable device, such as a spray can or similar containers.

In an embodiment, the present invention provides as composition comprising ingredients (a) and (b) as defined in the claims, for use in the treatment or the prevention of demodex infestation (demodicosis) and related diseases and conditions in a subject, wherein the use comprises the topical administration of said composition to the body part of the subject affected by demodicosis or demodicosis-related diseases or demodicosis- related disorders. Preferably, the composition of the present invention for use in a method of treatment as mentioned above is administered topically on skin, dermis, epidermis, ocular area and/or eyelid area, wherein the demodicosis or demodicosis-related diseases or demodicosis- related disorders is express.

The demodicosis-related disease are selected from blepharitis, rosacea, conjunctivitis, Meibomian gland dyspfunction, blepharo-conjunctivitis, eye burning, itchiness, irritation, redness, swelling, chalazion, dry eye and other disorders of the eyelid margins. The composition according to the invention is also useful for treating or relieving conditions deriving from postocular surgery, like corneal transplants, and for those undergoing cataract surgery.

The composition of the present invention, in any of the form or physical state above described, is a pharmaceutical composition, a composition for a medical device or a cosmetic composition.

The following examples are provided to illustrate practical embodiments of the invention.

### Comparative Example 1

### Formulation for 100g:

| **Components** | **Amount (%)** |
|---|---|
| Water | Up to 100 |
| Panthenol | 1.00 |
| Allantoine and/or aloe | 0.05 - 1.5 |
| Taurine | 1.80 |
| Tris-tromethamine | 0.235 |
| Acid boric | 0.30 |
| Cocamidopropyl betaine | 5.00 - 15.00 |
| PEG-40 hydrogenated castor oil, PPG-26 Buteth-26, Trideceth-9 | 1.00 - 10.00/1.5-15.00 |
| Terpinen-4-ol | 0.1-5.00 |
| *Chamaemelum nobile* essential oil | 0.10 |
| N-hydroxy methylglycinate sodium | 0.20 |
| EDTA | 0.10 |
| Helychrysum Glycolic extract | 0.20 |
| Aroma (* if present) | 0.10* |

### Example 2:

| **Components** | **Amount (%)** |
|---|---|
| Water | Up to 100 |
| Terpinen-4-ol | 1-5; 1.5-3.5 |
| Caprylyl/Capryl glucoside, sodium cocoyl glutamate, polyglyceryl-5-oleate, glyceryl caprylate(+ citric acid) | 1-5; 2-3.5 |
| Ethylhexylglycerin and phenethyl alcohol mixture | 0.1-2; 0.3-1.5 |
| Triethanolamine | 0.1-1.5; 0.2-0.7 |
| Hyaluronic acid Na salt - high MW | 0.01-0.5; 0.02-0.3 |
| EDTA .2Na | 0.01-0.1;0.02-0.07 |
| *Aloe vera gel* 1-1 polysaccharides 10% | 0.01-2; 0.02-1.5 |
| Panthenol | 0.001-2.5; 0.05-1.5 |
| Vitamin E acetate | 0.001-0.9; 0.005-0.6 |

### Biocompatibility tests

Biocompatibility tests concern the evaluation of the medical device in agreement with the guidelines stated in EN ISO 10993-1 Standard.

In the context of the present invention, according to the guidelines of the European Commission regarding the application of the Concil directive 93//EEC, the term "medical device" is defined as:
"Any instrument, apparatus, appliance, software, material or other article, whether used alone or in combination, including the software intended by its manufacturer to be used specifically for diagnostic and/or therapeutic purposes and necessary for its proper application, intended by the manufacturer to be used for human beings for the purpose of:
- diagnosis, prevention, monitoring, treatment or alleviation of disease,
- diagnosis, monitoring, treatment, alleviation of or compensation for an injury or handicap,
- investigation, replacement or modification of the anatomy or of a physiological process,
- control of conception,
and which does not achieve its principal intended action in or on the human body by pharmacological, immunological or metabolic means, but which may be assisted in its function by such means."

In deciding whether a product falls under the MDD, particular account shall be taken of the principal mode of action of the product.

Typically, the medical device function is achieved by physical means (including mechanical action, physical barrier, replacement of or support to organs or body functions ...).

The principal intended action of a medical device may be deduced from the scientific data regarding mechanism of action and the manufacturer's labelling and claims.

The ISO 10993 describes:
- the general principles governing the biological evaluation of medical devices within a risk management process;
- the general categorization of devices based on the nature and duration of their contact with the body;
- the evaluation of existing relevant data from all sources;
- the identification of gaps in the available data set on the basis of a risk analysis;
- the identification of additional data sets necessary to analyse the biological safety of the medical device;
- the assessment of the biological safety of the medical device.

The choice of tests and the data required in a biological evaluation, and their interpretation, shall take into account the chemical composition of the materials, including the conditions of exposure as well as the nature, degree, frequency and duration of exposure of the medical device or its constituents to the body, enabling the categorization of devices to facilitate the selection of appropriate tests. The rigour necessary in the biological evaluation is principally determined by the nature, degree, duration and frequency of the exposure and the hazards identified for the material.

For the medical devices according to the present invention in the form of foam or wipes the appropriate tests relate to:
Nature of Body Contact: skin
Contact duration: limited

On the basis of these considerations the tests concern:
- Maximization test on delayed hypersensitivity
- Cytotoxicity
- Skin Irritation Test
- Microbiology stability

### Cytotoxicity tests

The cytotoxicity assay for the formulation of Example 1 was performed on L929 fibroblasts cell cultures treated with scalar concentrations (1:2 dilutions from 5.0 mg/ml) of tested product. As positive control sodium dodecyl sulphate (SDS), a substance with well known cytotoxic effects, was used, while the negative reference was an internal standard with IC50 > 0.5 mg/ml (no cytotoxic substance).

A decrease in cell viability was observed at tested concentrations between 0.313 and 5.0 mg/ml with an IC50 = 1.164 mg/ml. An IC50 value > 0.5 mg/ml shows the absence of cytotoxic effects of tested product on fibroblasts cell culture.

The cytotoxicity assay for composition of Example 2 was performed on L929 fibroblasts cell cultures treated with scalar concentrations (1:2 dilutions from 5.0 mg/ml) of tested product. As positive control sodium dodecyl sulphate (SDS), a substance with well known cytotoxic effects, was used, while the negative reference was an internal standard with IC50 > 0.5 mg/ml (no cytotoxic substance).

No decrease in cell viability was observed at tested concentrations, therefore IC50 > 5.0 mg/ml. An IC50 value > 0.5 mg/ml shows the absence of cytotoxic effects of tested product on fibroblasts cell culture.

### Maximization test on delayed hypersensitivity

Delayed hypersensitivity test (Guinea Pig Maximisation Test, briefly GPMT) according to ISO 10993-10:2010 for the formula of Example 2 (test sample) was carried out aimed to identify its sensitising effects.

In the Guinea Pig Maximisation Test 15 guinea pigs were used, 10 of which were treated with the test item and 5 were used as a control group. GPMT consists of an induction phase and a challenge phase.

In the induction phase, guinea pigs were treated with 3 pairs of intradermal injections (each dose of 0.1 ml) thus subdivided:
- 1^{st} stable emulsion of Freud's Complete Adjuvant (FCA) in sodium chloride injection 50:50 (v:v);
- 2^{nd} test sample for treated animals, sodium chloride Injection for control animals;
- 3^{rd} test sample diluted 50:50 (v:v) with stable emulsion of FCA and sodium chloride Injection (50%) for treated animals, sodium chloride Injection diluted 50:50 (v:v) with stable emulsion of FCA and sodium chloride injection (50%) for control animals.

6 days after performing the intradermal injections- treated and control ones - a local application was performed on all the animals by massaging 0.5 ml of sodium lauryl sulphate at 10% in vaseline.
7 days after performing the intradermal injections, a patch of 8 cm2/animal of test sample was applied to the skin in 10 treated animals for 48 hours.

The same treatment was performed on control group using sodium chloride Injection.

In the challenge phase, 21 days after the beginning of the treatment on all the animals, both treated and control ones, the challenge phase was carried out by applying a patch of 8 cm2/animal of test sample on the right flank and 0.5 ml of sodium chloride Injection on the left flank. Bandaging was left for 24 hours. 48 and 72 hours after starting the challenge phase, the reactions of both treated and control animals were evaluated. The results were interpreted according to ISO 10993-10.

No abnormalities were observed in control animals.

No abnormalities were observed in treated animals with the test sample according the invention, thus the formulation of Example 2 (test sample) is not sensitizing.

### Skin Irritation Test

Skin irritation according to ISO 10993-10:2010 for the formulation of Example 2 (test sample) was carried out aimed to identify irritant effects

The skin irritation test was carried out through a semi-occlusive application; the test sample was applied on the intact skin of 3 rabbits, in the dorsal region both on the left and on the right side.

Each animal had the right caudal region and left cranial region treated with the test sample (25mm × 25 mm humidified patches). The right cranial region and the left caudal region were treated with a no irritant humidified gauze (25mm × 25 mm), used as control.

Reactions were evaluated 1 hour after the removal of the patches and were evaluated again at 24, 48 and 72 hours after exposure.

After 1 and 24 hours from the treatment, a slight erythema was observed in the treated sites of all animals. After 48 hours from the removal of the bandages, a slight erythema was detected in the treated sites of two animals. Nothing abnormal was detected in all control sites during the execution of the test.

Nothing abnormal was detected in all treated sites 72 hours after the treatment.

Primary skin irritation index (TREATED - CONTROL): 0.56, thus the formulation of Example 2 (test sample) is slightly irritant for skin (results, interpreted according to ISO 10993-10:2010).

### Microbiology stability

Challenge test for the formulation of Example 2 (test sample) was carried out aimed to evaluate microbiological stability. Challenge test consists in an attempt to reproduce in laboratory the microbial aggression a product suffers from in its life.

Phases of the challenge test are the following:
- preliminary microbial counter on the to-be-tested samples
- preparation of the micro-organisms for the inoculum
- inoculum of the samples
- check at preestablished intervals of the micro-organism's survival
- evaluation of the results.

The following selected strains of microorganisms were used:
- Gram - : Pseudomonas and Enterobacteriaceae
- Gram + : Staphylococcus and Micrococcus
- Yeasts : Candida
- Moulds : Aspergillus and Penicillium.

The efficacy of the preservative system is reckoned adequate when one notices a diminution through time of the micro-organisms number able to create colonies. In particular, for the test sample a considerable reduction by 2 days after the inoculum and an increasing diminution until 28th day after the inoculum was observed (UFC/g values for each bacterium versus time). According to the obtained results the formulation of Example 2 (test sample) is at low risk, thus it is an "appropriate preservative system".

### Efficacy test

The efficacy of the composition according to the invention is verified via experiments on the eyelashes from subjects with chronic blepharitis.

The subjects were not using topical or systemic antibacterial and anti-inflammatory medications.

Only epilated lashes of subjects containing live demodex are studied.

The sensitivity of the demodex to the treatment can vary between subjects.

Modifications in the movements and the morphology of the mites are observed. The movements of the demodex's legs, body and mouth are continuously observed under the microscope for a minimum of 4 hours (240 minutes). The survival time is defined as the time when the treatment was added to the time when the movement stopped. Survival times of the different groups of treatments were compared.

When demodex are alive they cling to the lashes and when die they tend to detach from the eyelashes.

When the composition according to the invention is used, independently of the presence of demodex, eyelashes detritus are eliminated or dissolved, leaving the eyelashes cleaner.

Time of killing of larvae was significantly lower than adult time.

Application of the composition according to the invention decreases significantly the mites which may be present on the eyelashes of subjects with blepharitis and removes any debris from the lashes.

## Claims

1. A composition comprising a mixture which comprises or, alternatively, consists of:
(a) terpinen-4-ol, and
(b) a mixture comprising or, alternatively, consisting of: caprylyl/capryl glucoside (b5), sodium cocoyl glutamate (b6), polyglyceryl-5 oleate (b7), glyceryl caprylate (b8);
and, optionally, at least one additive and/or an excipient of pharmaceutical or cosmetic grade.

2. The composition according to claim 1, wherein the amount of terpinen-4-ol is comprised from 0.01% to 10% by weight with respect to the total weight of the composition.

3. The composition according to claim 2, wherein the amount of terpinen-4-ol is comprised from 0.1% to 5% by weight with respect to the total weight of the composition.

4. The composition according to any one of the preceding claims, wherein the weight ratio between ingredient (b) and terpinen-4-ol (a) is from 0.01:1 to 10:1.

5. The composition according to claim 4, wherein the weight ratio between ingredient (b) and terpinen-4-ol (a) is from 0.1:1 to 10:1, preferably from 1:1 to 7:1.

6. The composition according to any one of the preceding claims, wherein said composition further comprises a surfactant (c) which is selected from the group comprising or, alternatively, consisting of: cocamidopropyl betaine, lauryl ether sulfate, lauryl ether sulfosuccinate, and mixtures thereof.

7. The composition according to claim 6, wherein said surfactant (c) is in an amount comprised from 1% to 25% by weight, with respect to the total weight of the composition; preferably in an amount comprised from 8% to 15% by weight.

8. The composition according to any one of the preceding claims, wherein the composition is in a form or physical state of a liquid, solid or gel.

9. The composition according to claim 8, wherein said composition is in the form or physical state of a liquid and wherein said at least one ingredient (b) is said mixture comprising or, alternatively, consisting of caprylyl/capryl glucoside (b5), sodium cocoyl glutamate (b6), polyglyceryl-5 oleate (b7), glyceryl caprylate (b8).

10. The composition according to any one of the preceding claims, wherein said composition is for use in a method of treatment or prevention of demodex infestation (demodicosis), of a demodicosis-related disease or demodicosis-related disorder in a subject in the need thereof, wherein said composition is used by topical administration, wherein the demodicosis-related disease and/or demodicosis-related disorder is selected in a group comprising or, alternatively, consisting of: blepharitis, rosacea, conjunctivitis, Meibomian gland dysfunction, blepharo-conjunctivitis, eye burning, itchiness, irritation, redness, swelling, chalazion, dry eye and other disorders of the eyelid margins.

11. A solid substrate comprising the composition according to any one of claims 1-9, wherein said composition in the form or physical state of a liquid is absorbed on, or impregned by, the solid substrate.

12. The solid substrate according to claim 11 wherein said solid substrate is a wipe or a tissue.

13. A method for the preparation of the composition according to any one of claims 1-9, comprising a step of:
(i) mixing the components (a) and (b) in the presence of water or a water-miscible solvent.

14. The method according to claim 13 wherein said water or water-miscible solvent in step (i) is present in an amount from 50% to 95% by weight with respect to the total weight of the mixture of step (i).

## Patentansprüche

1. Zusammensetzung, umfassend eine Mischung, die umfasst oder alternativ dazu besteht aus:
(a) Terpinen-4-ol und
(b) eine/r Mischung umfassend oder alternativ dazu bestehend aus: Caprylyl/Capryl-Glucosid (b5), Natrium-Cocoyl-Glutamat (b6), Polyglyceryl-5-Oleat (b7), Glyceryl-Caprylat (b8);
und optional mindestens einen/m Zusatzstoff und/oder einen/m Hilfsstoff pharmazeutischer oder kosmetischer Qualität.

2. Zusammensetzung nach Anspruch 1, wobei die Menge an Terpinen-4-ol im Bereich von 0,01 bis 10 Gew.- %, in Bezug auf das Gesamtgewicht der Zusammensetzung, beträgt.

3. Zusammensetzung nach Anspruch 2, wobei die Menge an Terpinen-4-ol im Bereich von 0,1 bis 5 Gew.- %, in Bezug auf das Gesamtgewicht der Zusammensetzung, beträgt.

4. Zusammensetzung nach einem der vorhergehenden Ansprüche, wobei das Gewichtsverhältnis vom Inhaltsstoff (b) zu Terpinen-4-ol (a) von 0,01: 1 bis 10:1 beträgt.

5. Zusammensetzung nach Anspruch 4, wobei das Gewichtsverhältnis vom Inhaltsstoff (b) zu Terpinen-4-ol (a) von 0,1: 1 bis 10: 1, vorzugsweise von 1:1 bis 7:1 beträgt.

6. Zusammensetzung nach einem der vorhergehenden Ansprüche, wobei die Zusammensetzung ferner ein Tensid (c) umfasst, das aus der Gruppe ausgewählt ist, die umfasst oder alternativ dazu besteht aus: Cocamidopropylbetain, Laurylethersulfat, Laurylethersulfosuccinat und Mischungen davon.

7. Zusammensetzung nach Anspruch 6, wobei das Tensid (c) in einer Menge im Bereich von 1 bis 25 Gew.- %, in Bezug auf das Gesamtgewicht der Zusammensetzung, vorliegt; vorzugsweise in einer Menge im Bereich von 8 bis 15 Gew.- %.

8. Zusammensetzung nach einem der vorhergehenden Ansprüche, wobei die Zusammensetzung in einer Form oder einem physikalischen Zustand einer Flüssigkeit, eines Feststoffs oder eines Gels vorliegt.

9. Zusammensetzung nach Anspruch 8, wobei die Zusammensetzung in Form oder im physikalischen Zustand einer Flüssigkeit vorliegt und wobei der mindestens eine Inhaltsstoff (b) die Mischung ist, die Caprylyl/Capryl-Glucosid (b5), Natrium-Cocoyl-Glutamat (b6), Polyglyceryl-5-Oleat (b7), Glyceryl-Caprylat (b8) umfasst oder alternativ dazu daraus besteht.

10. Zusammensetzung nach einem der vorhergehenden Ansprüche, wobei die Zusammensetzung zur Verwendung in einem Verfahren zur Behandlung oder Vorbeugung von Demodex-Befall (Demodikose), einer Demodikose-bedingten Erkrankung oder Demodikose-bedingten Störung bei einem Subjekt, das diese benötigt, bestimmt ist, wobei die Zusammensetzung durch topische Verabreichung verwendet wird, wobei die Demodikose-bedingte Erkrankung und/oder Demodikose-bedingte Störung aus einer Gruppe ausgewählt ist, die umfasst oder alternativ dazu besteht aus: Blepharitis, Rosacea, Konjunktivitis, Meibom-Drüsen-Dysfunktion, Blepharon-Konjunktivitis, Augenbrennen, Juckreiz, Reizung, Rötung, Schwellung, Chalazion, trockenem Auge und anderen Störungen der Augenlidränder.

11. Festes Substrat, umfassend die Zusammensetzung nach einem der Ansprüche 1-9, wobei die Zusammensetzung in Form oder im physikalischen Zustand einer Flüssigkeit auf dem festen Substrat absorbiert oder von diesem imprägniert ist.

12. Festes Substrat nach Anspruch 11, wobei das feste Substrat ein Tuch oder ein Gewebe ist.

13. Verfahren zur Vorbereitung der Zusammensetzung nach einem der Ansprüche 1-9, umfassend einen Schritt zum:
(i) Mischen der Komponenten (a) und (b) in Gegenwart von Wasser oder eines mit Wasser mischbaren Lösungsmittels.

14. Verfahren nach Anspruch 13, wobei das Wasser oder das mit Wasser mischbare Lösungsmittel in Schritt (i) in einer Menge im Bereich von 50 bis 95 Gew.- %, in Bezug auf das Gesamtgewicht der Mischung aus Schritt (i), vorhanden ist.

## Revendications

1. Composition, comprenant un mélange qui comprend ou, alternativement, consiste en :
(a) terpinen-4-ol, et
(b) un mélange comprenant ou, alternativement, consistant en : caprylyl/capryl glucoside (b5), cocoyl glutamate de sodium (b6), polyglycéryl-5 oléate (b7), caprylate de glycéryle (b8) ;
et, éventuellement, au moins un additif et/ou un excipient de qualité pharmaceutique ou cosmétique.

2. Composition selon la revendication 1, dans laquelle la quantité de terpinen-4-ol est comprise entre 0,01 % et 10 % en poids par rapport au poids total de la composition.

3. Composition selon la revendication 2, dans laquelle la quantité de terpinen-4-ol est comprise entre 0,1 % et 5 % en poids par rapport au poids total de la composition.

4. Composition selon l'une quelconque des revendications précédentes, dans laquelle le rapport pondéral entre l'ingrédient (b) et le terpinen-4-ol (a) est de 0,01 : 1 à 10:1.

5. Composition selon la revendication 4, dans laquelle le rapport pondéral entre l'ingrédient (b) et le terpinen-4-ol (a) est de 0,1 : 1 à 10:1, de préférence de 1:1 à 7:1.

6. Composition selon l'une quelconque des revendications précédentes, dans laquelle ladite composition comprend en outre un tensioactif (c) qui est choisi dans le groupe comprenant ou, alternativement, consistant en : cocamidopropyl bétaïne, lauryl éther sulfate, lauryl éther sulfosuccinate, et des mélanges de ceux-ci.

7. Composition selon la revendication 6, dans laquelle ledit tensioactif (c) est en une quantité comprise entre 1 % et 25 % en poids, par rapport au poids total de la composition ; de préférence en une quantité comprise entre 8 % et 15 % en poids.

8. Composition selon l'une quelconque des revendications précédentes, dans laquelle la composition est sous la forme ou dans l'état physique d'un liquide, d'un solide ou d'un gel.

9. Composition selon la revendication 8, dans laquelle ladite composition est sous la forme ou l'état physique d'un liquide et dans laquelle ledit au moins un ingrédient (b) est ledit mélange comprenant ou, alternativement, consistant en caprylyl/capryl glucoside (b5), cocoyl glutamate de sodium (b6), polyglycéryl-5 oléate (b7), caprylate de glycéryle (b8).

10. Composition selon l'une quelconque des revendications précédentes, dans laquelle ladite composition est destinée à être utilisée dans une méthode de traitement ou de prévention de l'infestation par le démodex (démodécie), d'une maladie liée à la démodécie ou d'un trouble lié à la démodécie chez un sujet qui en a besoin, dans laquelle ladite composition est utilisée par administration topique, dans laquelle la maladie liée à la démodécie et/ou le trouble lié à la démodécie est sélectionné dans un groupe comprenant ou, alternativement, consistant en : blépharite, rosacée, conjonctivite, dysfonctionnement des glandes de Meibomius, blépharon-conjonctivite, brûlure oculaire, démangeaisons, irritation, rougeur, gonflement, chalazion, sécheresse oculaire et autres troubles des bords des paupières.

11. Substrat solide, comprenant la composition selon l'une quelconque des revendications 1 à 9, dans lequel ladite composition sous la forme ou l'état physique d'un liquide est absorbée sur, ou imprégnée par, le substrat solide.

12. Substrat solide selon la revendication 11, dans lequel ledit substrat solide est une lingette ou un tissu.

13. Procédé de préparation de la composition selon l'une quelconque des revendications 1 à 9, comprenant une étape de :
(i) mélanger les composants (a) et (b) en présence d'eau ou d'un solvant miscible à l'eau.

14. Procédé selon la revendication 13, dans lequel ladite eau ou ledit solvant miscible à l'eau dans l'étape (i) est présent en une quantité de 50 % à 95 % en poids par rapport au poids total du mélange de l'étape (i).
